Europäisches Patentamt

(19) European Patent Office

Office européen des brevets

(11) EP 0 673 643 B1

## (12) FASCICULE DE BREVET EUROPEEN

(45) Date de publication et mention
de la délivrance du brevet:
**24.07.1996 Bulletin 1996/30**

(51) Int. Cl.⁶: $A61K\ 7/48$, $A61K\ 7/06$

(21) Numéro de dépôt: **95400295.2**

(22) Date de dépôt: **13.02.1995**

(54) **Composition cosmétique contenant en association une superoxyde dismutase et une porphyrine**

Kosmetisches Mittel enthaltend einen Assoziat aus Superoxyd-Dismutase und Porphyrine

Cosmetic composition containing an association of superoxide dismutase and porphyrine

(84) Etats contractants désignés:
**AT BE CH DE ES FR GB IT LI NL SE**

(30) Priorité: **21.03.1994 FR 9403271**

(43) Date de publication de la demande:
**27.09.1995 Bulletin 1995/39**

(73) Titulaire: **L'OREAL**
**F-75008 Paris (FR)**

(72) Inventeurs:
• **Nguyen, Quang Lan**
**F-92160 Antony (FR)**

• **Colin, Christian**
**F-75020 Paris (FR)**

(74) Mandataire: **Lhoste, Catherine**
**L'OREAL,**
**90 rue du Général Roguet**
**F-92583 Clichy Cédex (FR)**

(56) Documents cités:
**EP-A- 0 504 040**      WO-A-92/19224
**WO-A-94/02484**      FR-A- 2 287 899

## Description

La présente invention a pour objet des compositions cosmétiques et/ou dermatologiques contenant une superoxyde dismutase (SOD) en association avec une porphyrine.

De telles compositions utilisées par voie topique permettent notamment de lutter contre le vieillissement cutané et/ou de protéger la peau contre les effets des radicaux libres induits par exemple par les polluants atmosphériques et/ou par le rayonnement ultra-violet. On sait en effet que la toxicité des polluants atmosphériques, notamment des polluants gazeux comme le dioxyde de soufre, l'ozone et les oxydes d'azote, est liée à leur activité d'initiateurs de radicaux libres qui sont la source de phénomènes d'oxydation provoquant chez les êtres vivants des dommages cellulaires. Les cellules d'organes qui sont en contact direct et permanent avec le milieu extérieur, comme la peau, le cuir chevelu et certaines muqueuses, sont particulièrement sensibles à ces effets des polluants gazeux, qui se traduisent notamment par un vieillissement accéléré de la peau, avec un teint manquant d'éclat et une formation précoce de rides et de ridules, et aussi par une diminution de la vigueur et un aspect terne des cheveux.

On sait que les superoxyde dismutases sont des enzymes capables d'induire la dismutation des ions superoxydes, suivant la réaction:

$$2\ O°^{-}_{2} + 2\ H^{+} \rightarrow H_2O_2 + O_2$$

On connaît de nombreuses superoxyde dismutases. Par exemple, on a déjà décrit des superoxyde dismutases extraites d'érythrocytes de boeuf (Markovitz, J. Biol. Chem. 234, p. 40, 1959) et des superoxyde dismutases extraites d'*Escherichia coli* (Keele et Fridovich, J. Biol. Chem., 245, p. 6176, 1970). Dans le document FR-A-2.225.443, sont décrites des superoxyde dismutases extraites de souches bactériennes marines, ainsi que leur procédé de préparation.

Les superoxyde dismutases permettent en particulier de protéger la peau et les cheveux notamment en maintenant l'intégrité de la structure kératinique naturelle, comme le décrit par exemple le document FR-A-2.287.899. En outre, les superoxyde dismutases améliorent la respiration cellulaire cutanée et maintiennent ou améliorent les qualités de la peau telles que la douceur au toucher, la souplesse et l'élasticité. Leur présence dans des compositions pour cheveux permet aussi le maintien ou l'amélioration de l'état du cuir chevelu, tout en protégeant également la peau des mains de la personne qui applique ces compositions.

Les superoxyde dismutases protègent aussi la peau contre les phénomènes d'inflammation causés par les rayonnements ultra-violets ainsi que contre le vieillissement accéléré de la peau notamment sous l'influence de tels rayonnements.

Grâce à ces différentes propriétés, les superoxyde dismutases sont utilisables dans des compositions cosmétiques pour la peau, les cheveux et/ou les muqueuses, ainsi que dans des compositions pharmaceutiques à usage dermatologique.

Les superoxyde dismutases agissent contre les radicaux libres oxygénés. L'ion superoxyde $O°^{-}_{2}$ (oxygène actif) est un ion-radical dont l'instabilité et la réactivité en font un composé toxique, car il engendre, notamment en présence d'ions métalliques, des radicaux libres hydroxyle (°OH) hautement nocifs. Les superoxyde dismutases exercent un effet protecteur notamment en piégeant les ions superoxydes et constituent donc un système biologique de défense contre les effets nocifs des radicaux libres oxygénés.

Malheureusement, les superoxyde dismutases seules dans une composition cosmétique présentent une efficacité insuffisante pour piéger complètement les radicaux libres oxygénés. Pour résoudre ce problème, on a déjà envisagé de l'associer à un séquestrant du type phosphonique (voir FR-A-2.675.997).

La demanderesse a maintenant trouvé un nouveau composé à associer à la superoxyde dismutase pour renforcer son action antiradicalaire.

De façon plus précise, l'invention a pour objet une composition cosmétique et/ou dermatologique caractérisée par le fait qu'elle comprend au moins une superoxyde dismutase et au moins une porphyrine.

Certes, il est connu par le document JP-A-02019311 d'utiliser l'hémine et l'hématine pour protéger la peau de la lumière du soleil et pour prévenir le vieillissement de la peau, et il est connu par le document FR-A-2.693.904 d'utiliser des chlorophylles pour restructurer la peau et pour prévenir aussi le vieillissement de la peau. Mais ces documents ne décrivent nullement ni ne suggèrent d'associer une porphyrine avec une superoxyde dismutase en vue de renforcer, avec une synergie, l'activité de cette dernière.

Les superoxyde dismutases auxquelles s'applique l'invention sont les enzymes SOD et tout produit équivalent ayant une activité analogue à celle des superoxyde dismutases, à savoir toute enzyme naturelle qui peut catalyser la réaction de dismutation ci-dessus indiquée ainsi que tout produit ayant cette activité, ce qui inclut notamment les enzymes modifiées telles que les SOD modifiées par greffage de polyoxyalkylènes, de polyéthylèneglycols, de polysaccharides ou de groupes acylés, ainsi que les substances contenant de tels produits.

Les enzymes SOD, qui sont des métalloprotéines dont le constituant métallique est le fer, le manganèse, le cuivre et/ou le zinc, peuvent être d'origines diverses. On peut notamment citer les SOD d'origine animale (par exemple bovine ou porcine), humaine (par exemple placentaire ou sanguine), végétale (par exemple extraites de champignons, algues, épinards,...), ou les SOD extraites de microorganismes (bactéries ou levures), ou encore les SOD recombinantes obtenues par génie génétique.

Parmi les exemples de SOD d'origine bovine, on peut citer en particulier la SOD de type Cu-Zn qui a été purifiée jusqu'à homogénéité et approuvée pour des applications cliniques (New Trends in Allergy, I. Ring et al., Ed. Springer Verlag 1986).

Parmi les SOD obtenues à partir de cultures de bactéries, levures ou cellules animales, on peut citer la SOD Cu-Zn humaine recombinante commercialisée par la Société Ube Industries Ltd.

Parmi les exemples de SOD extraites de bactéries, on peut citer en particulier celles extraites d'*Escherichia coli* ; parmi les superoxyde dismutases extraites de champignons, on peut citer en particulier celles extraites de *Pleurotus olearius* ; parmi les superoxyde dismutases extraites du sang, on peut citer les érythrocupréines.

On peut citer également les superoxyde dismutases extraites de souches bactériennes marines, telles que par exemple des souches de *Photobacterium phosphoreum*, *Photobacterium leiognathi* ou *Photobacterium sepia*. Parmi les diverses souches utilisables, on peut citer les souches de *Photobacterium phosphoreum* n° ATCC 11040, de *Photobacterium leiognathi* n° ATCC 25521, de *Photobacterium sépia* n° ATCC 15709, d'*Escherichia coli* n° ATCC 15224 et de *Pleurotus olearius* Gillet (Laboratoire de Cryptogamie de Paris).

Les SOD utilisées selon l'invention peuvent être préparées par application des méthodes déjà décrites, par exemple dans l'article de Keele et al cité ci-dessus ainsi que dans Eur. J. Rheumatol. and Inflammation, 4, 173-182 (1982).

Les SOD extraites de souches bactériennes marines peuvent être préparées selon le procédé décrit dans la demande FR-A-2.225.443 ci-dessus mentionnée.

Les SOD utilisées selon l'invention peuvent aussi être des SOD modifiées notamment selon l'enseignement du document "International Conference on Medical, Biochemical and Chemical Aspects of Free Radicals" (1988 Kyoto) p. 317, article de H. Morimoto, ou selon l'article de Ando Yukio p. 318 (même source), ou encore selon les documents JP-A-01250304 (Kanebo) et JP-A-02273176. On peut citer également les SOD modifiées décrites dans les demandes de brevet européen EP-A-424 033 et EP-A-426 488.

Les SOD utilisées selon l'invention peuvent être en outre employées sous forme stabilisée selon les techniques connues, par exemple à l'aide de phosphate, en présence de chlorure de métal alcalin et de saccharose ; voir par exemple le document FR-A-2.634.125.

Les porphyrines utilisables selon l'invention sont en particulier des porphyrines à transfert de charge comportant un métal bivalent choisi parmi le fer, le magnésium, le cuivre, le zinc et le manganèse. A titre d'exemple, les porphyrines sont choisies parmi la chlorophylle, notamment la chlorophylle A, la chlorophylline, l'hémoglobine, ainsi que les extraits végétaux et/ou animaux contenant de la chlorophylle ou de l'hémoglobine.

Comme chlorophylle, on peut citer la chlorophylle A vendue par la société Sigma.

Comme extraits végétaux contenant de la chlorophylle, on peut citer par exemple l'extrait de thé, vendu sous la dénomination "Sunphenon" par la société Nikkol, l'extrait de romarin, vendu sous la dénomination "Rosmanox Powder" par la société Marcus, l'extrait aromatisé de reine des prés, vendu sous la dénomination Supextrat de reine des prés par la société Sochibo, l'extrait de thym, vendu par la société Sarpap, l'extrait de mélisse, vendu par la société Sarpap et l'extrait d'épinard, vendu sous la dénomination "Spinach Acetone Powder" par la société Sigma.

Comme hémoglobine, on peut citer l'hémoglobine bovine vendue par la société Sigma.

Comme chlorophylline, on peut citer celle vendue par la société Wackherr.

L'association de l'invention trouve principalement une application dans la réalisation de compositions cosmétiques et/ou dermatologiques destinées à prévenir et/ou lutter contre le vieillissement cutané et/ou à protéger la peau, les cheveux et/ou les muqueuses contre les effets nocifs et/ou inesthétiques provoqués par les radicaux libres induits notamment par les polluants atmosphériques et/ou par le rayonnement ultra-violet. L'invention a donc également pour objet de telles compositions.

Dans les compositions de l'invention, la concentration de la SOD peut être choisie par exemple dans la gamme de 40 à 5000 unités enzymatiques de SOD pour 100 g de composition, et notamment de 250 à 1500 unités. L'unité enzymatique de SOD est définie par Mc Cord et Fridovitch, J. Biol. Chem. 244, 6049 (1969).

La porphyrine est généralement présente à raison de 0,001 % à 1 % en poids, et notamment de 0,025 % à 0,1 % en poids par rapport au poids total de la composition.

Les compositions selon l'invention peuvent renfermer l'association de SOD et de porphyrine soit à titre d'ingrédient actif principal, soit à titre de protecteur contre l'oxydation des autres ingrédients. Dans le cas où les ingrédients oxydables à protéger subissent une décomposition accélérée en présence des fibres kératiniques et/ou de la peau et/ou des muqueuses, l'association de SOD et de porphyrine peut être conservée seule, en solution aqueuse diluée ou concentrée, ou sous forme de complexe ou de lyophilisat, et n'être ajoutée aux autres ingrédients de la composition qu'au moment de l'emploi.

De même, lorsque l'association de SOD et de porphyrine est utilisée dans le but de maintenir ou d'améliorer les qualités de la peau et/ou des cheveux et/ou des muqueuses, cette association peut n'être ajoutée à la composition qu'au moment de l'emploi.

Les compositions selon l'invention peuvent donc être conditionnées en plusieurs parties avec une première partie contenant la SOD et la porphyrine, et une deuxième partie contenant les autres ingrédients de la composition.

Les compositions pour la peau selon l'invention peuvent contenir, outre l'association binaire SOD et porphyrine, des ingrédients actifs ou excipients utilisés de façon usuelle dans les formulations cosmétiques ou dermatologiques, tels que des tensio-actifs, des colorants, des parfums, des agents conservateurs, des émulsionnants, des véhicules liquides tels que l'eau, des corps gras destinés à constituer la phase grasse d'émulsions (telles que laits ou crèmes), des résines, etc.

Les composés destinés à constituer une phase grasse sont par exemple des huiles minérales, animales, végétales ou synthétiques, des huiles siliconées et/ou fluorées, des cires, des alcools gras ou encore des acides gras.

Parmi les huiles minérales, on peut citer par exemple l'huile de vaseline, et parmi les huiles synthétiques, les palmitates d'éthyle et d'isopropyle, les myristates d'alkyle tels que le myristate d'isopropyle, de butyle, ou de cétyle, le stéarate d'hexyle, les triglycérides des acides octanoïque et décanoïque (par exemple le produit vendu sous la dénomination "Miglyol" par la Société Dynamit-Nobel), le ricinoléate de cétyle, l'octanoate de stéaryle (huile de purcellin) et l'octanoate de polyisobutène hydroxylé.

Parmi les huiles végétales, on peut citer par exemple l'huile d'amande douce, l'huile d'avocat, l'huile de coco, l'huile de germe de blé, l'huile de maïs, l'huile de ricin, l'huile d'olive, l'huile de palme, l'huile de sésame, l'huile de soja, l'huile d'argan, l'huile d'onagre, l'huile de bourrache, les huiles essentielles et les cires végétales et la cire d'abeille.

Parmi les alcools gras, on peut citer l'alcool cétylique, l'alcool stéarylique, l'alcool myristique, l'alcool hydroxystéarylique, l'alcool oléique, l'alcool isostéarylique, l'alcool laurylique, l'alcool hexadécylique, l'alcool ricinoleylique, l'alcool béhénylique, l'alcool érucylique et le 2-octyldodécanol.

Parmi les acides gras, on peut mentionner l'acide stéarique, l'acide myristique, l'acide palmitique, l'acide oléique, l'acide linoléique, l'acide laurique, l'acide isostéarique, l'acide hydroxystéarique, l'acide linolénique, l'acide ricinoléique, l'acide arachidique, l'acide behénique, l'acide érucique et les acides lanoliniques.

Les compositions selon l'invention destinées à une application topique sont notamment des solutions ou dispersions du type lotion ou sérum, des émulsions de consistance liquide ou semi-liquide du type lait, obtenues par dispersion d'une phase grasse dans une phase aqueuse (H/E) ou inversement (E/H), ou des suspensions ou émulsions de consistance molle du type crème ou gel, ou encore des microgranulés, ou des dispersions vésiculaires de type ionique et/ou non ionique.

Ces compositions sont préparées selon les méthodes usuelles. Elles constituent notamment des crèmes de nettoyage, de protection ou de soin pour le visage, pour les mains ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, ou des compositions désodorisantes contenant un agent bactéricide.

Les compositions selon l'invention peuvent également consister en des préparations solides constituant des savons ou des pains de nettoyage.

Les compositions peuvent aussi être conditionnées sous forme de composition pour aérosol contenant également un agent propulseur sous pression.

Les compositions pour cheveux selon l'invention peuvent être présentées sous forme de solutions aqueuses, alcooliques ou hydroalcooliques, ou sous forme de crèmes, de gels, d'émulsions, de mousses ou encore sous forme de composition pour aérosol contenant également un agent propulseur sous pression.

Outre les ingrédients actifs classiques, elles peuvent renfermer divers adjuvants habituellement présents dans ces compositions pour cheveux par exemple des véhicules liquides ou sous forme de gels, des parfums, des colorants, des agents conservateurs, des agents épaississants, etc.

Les quantités des différents constituants des compositions selon l'invention sont celles classiquement utilisées dans les domaines considérés.

L'association selon l'invention peut être incorporée, à titre d'ingrédient principal ou secondaire, dans diverses compositions pour soins capillaires constituant par exemple des crèmes, lotions, gels, sérums ou mousses pour le soin du cuir chevelu, des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent être notamment des shampooings contenant, outre une superoxyde dismutase et une porphyrine, un détergent cationique, anionique ou non ionique ; des compositions de teinture, y compris des shampooings colorants, qui contiennent des colorants ou des précurseurs de colorant usuels ; des compositions pour le premier temps (temps de réduction) d'une déformation des cheveux, contenant des dérivés réducteurs tels que mercaptans, sulfites, etc. ; des compositions pour le ralentissement de la chute des cheveux et pour favoriser la repousse des cheveux, contenant des composés tels que le "Minoxidil" (2,4-diamino-6-pipéridino-pyrimidine-3-oxyde) et ses dérivés, le Diazoxide (7-chloro-3-méthyl-1,2,4-benzothiadiazine 1,1-dioxyde) et le "Phenytoïn" (5,5-diphényl imidazolidine 2,4-dione).

Les compositions cosmétiques de l'invention peuvent aussi être à usage bucco-dentaire, par exemple une pâte dentifrice. Dans ce cas, les compositions peuvent contenir des adjuvants et additifs usuels pour les compositions à usage buccal et notamment des agents tensio-actifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Les compositions cosmétiques selon l'invention peuvent être aussi bien des compositions prêtes à l'emploi que des concentrés devant être dilués avant l'utilisation. Les compositions pouvant être présentées sous forme de concentrés sont par exemple des shampooings ou des compositions pour bains.

L'invention a aussi pour objet une utilisation en association, d'au moins une superoxyde dismutase et d'au moins une porphyrine comme ingrédients actifs dans ou pour la fabrication d'une composition cosmétique et/ou dermatologique pour prévenir et/ou lutter contre le vieillissement cutané et/ou pour protéger la peau, les cheveux et/ou les muqueuses contre les effets nocifs et/ou inesthétiques provoqués par les radicaux libres. En particulier, cette composition est celle définie ci-dessus.

La présente invention a en outre pour objet un procédé de traitement cosmétique caractérisé par le fait que l'on applique sur la peau, sur les cheveux, et/ou sur les muqueuses, une composition telle que décrite ci-dessus contenant au moins une superoxyde dismutase et au moins une porphyrine.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux, application d'une lotion pour cheveux sur cheveux mouillés, shampooings, ou encore application de dentifrice sur les gencives.

Le procédé de traitement cosmétique de l'invention est mis en oeuvre de façon à appliquer une quantité efficace de l'association de SOD et de porphyrine, c'est à dire une quantité suffisante pour obtenir l'effet de protection recherché.

Ce procédé de traitement cosmétique est destiné en particulier à maintenir la structure kératinique de la peau ou des cheveux, de façon à éviter la dégradation de ceux-ci et les effets inesthétiques d'une telle dégradation sous l'influence des radicaux libres induits notamment par les polluants atmosphériques, à maintenir ou améliorer les qualités de la peau (douceur, souplesse, élasticité), des cheveux ou des muqueuses, à protéger la peau ou les cheveux contre les effets nocifs des rayons ultra-violets, et en particulier à traiter ou prévenir le vieillissement précoce de la peau.

Les exemples suivants illustrent l'invention. Dans ces exemples, les proportions indiquées sont des pourcentages en poids.

Exemples de formulation :

Exemple 1 : Emulsion Huile dans eau

| | |
|---|---|
| SOD (vendue par la Société Pentapharm et titrant 3,13 u/mg), q.s.p. 600 unités | 0,20 |
| Extrait de thé vert (Sunphenon de la société Nikkol) | 0,05 |
| Polyéthylèneglycol polyoxyéthyléné à 50 moles d'oxyde d'éthylène | 1,50 |
| Monostéarate de diglycéryle | 1,50 |
| Huile de vaseline | 24,00 |
| Alcool cétylique | 2,50 |
| Triéthanolamine | q.s. pH 7 |
| Eau | q.s.p 100 % |

Cette émulsion, préparée de manière usuelle, peut être utilisée notamment comme crème de jour.

Exemple 2 : Emulsion Eau dans l'huile

| | |
|---|---|
| SOD (vendue par la Société Bio-technologie et titrant 8037 u/mg), q.s.p. 1000 unités | 0,00012 |
| Chlorophylline (vendue par Wackherr) | 0,1 |
| Sesquiisostéarate de polyglycéryle | 4,0 |
| Cire d'abeille blanche | 0,5 |
| Stéarate de magnésium | 1,5 |
| Stéarate d'aluminium | 1,0 |
| Huile de ricin hydrogénée polyoxyéthylénée (à 7 moles d'oxyde d'éthylène) | 3,0 |
| Palmitate d'isopropyle | 10,0 |
| Perhydrosqualène | 15,0 |
| Eau | q.s.p. 100 % |

Cette émulsion, préparée de manière usuelle, est utilisable notamment comme crème de soin.

Exemple 3 : Dispersion vésiculaire

| | |
|---|---|
| Amphiphile non ionique* | 0,9 |
| Acylglutamate de sodium HS21 (vendu par la société Ajinomoto) | 0,1 |
| Glycérine | 3,0 |
| SOD (selon exemple 1) q.s.p. 250 unités | 0,08 |
| Extrait d'épinard (vendu par la société Sigma) | 0,1 |
| Perhydrosqualène | 10,0 |
| Parahydroxybenzoate de méthyle | 0,2 |
| Acide polyacrylique réticulé (Carbopol 940 vendu par la Société Goodrich) | 0,4 |
| Triéthanolamine | q.s. pH = 7 |
| Eau | q.s.p. 100 % |

* L'amphiphile non ionique est un mélange de produits répondant à la formule suivante :

$$C_{12}H_{25}[OC_2H_3(R)\text{-}O\text{-}C_3H_5(OH)\text{-}O]_n\text{-}H$$

dans laquelle :
n, représentant le nombre statistique de motifs, est égal à 2,7,
les groupements $-OC_2H_3(R)-$ représentent des radicaux :

$$\begin{array}{cc} -O\text{-}CH\text{-}CH_2\text{-} & \text{et } -O\text{-}CH_2\text{-}CH\text{-} \\ | & | \\ R & R \end{array}$$

et les groupements $-C_3H_5(OH)\text{-}O-$ représentent des radicaux :

$$\begin{array}{cc} -CH_2\text{-}CH\text{-}O & \text{et } -CH\text{-}CH_2\text{-}O\text{-} \\ | & | \\ CH_2OH & CH_2OH \end{array}$$

avec R représentant les radicaux $C_{14}H_{29}$ et $C_{16}H_{33}$, statistiquement en quantités molaires égales.

Le produit vendu sous la dénomination "Acylglutamate HS21" est un stéarylglutamate disodique.
Cette dispersion vésiculaire est préparée de la façon suivante :

- le composé amphiphile non ionique est ajouté à l'acylglutamate à une température de 100°C ;
- la température est abaissée à 90°C et on ajoute à cette température la glycérine, l'extrait d'épinard et l'eau ;
- le mélange est refroidi à 50°C, la SOD ajoutée, puis on homogénéise 2 fois 4 minutes à l'aide d'un homogénéiseur Virtis 60 (à 40.000 tours par minute) ;

7

- le produit obtenu est refroidi rapidement jusqu'à la température ambiante et dilué avec 20 g d'eau. La phase huileuse (perhydrosqualène et parahydroxybenzoate de méthyle) est ajoutée puis on homogénéise 2 fois 4 minutes à 40.000 tours par minute ;
- le gel de Carbopol (Carbopol 940 et eau q.s.p. 100 g) est dispersé pendant 30 secondes à 10.000 tours par minute puis l'ensemble est neutralisé par la triéthanolamine.

On obtient une crème lisse et brillante, utilisable notamment comme crème de soin.

Test d'inhibition de la production d'éthylène :

Dans une boîte de Pétri de diamètre 32 mm, sont introduits dans l'ordre suivant :

- 1,4 ml de tampon phosphate 50 mM (pH = 7,4),
- 100 µl de solution 200 mM de méthionine,
- 100 µl de solution 4 mM de chlorure ferrique,
- 100 µl du produit à tester,
- 100 µl de solution 4 mM d'E.D.T.A.(acide éthylènediamine tétracétique),
- 100 µl de solution 400 mM de N.A.D.H. (nicotinamide adénine dinucléotide, forme réduite),
- 100 µl de solution 2 mM de riboflavine.

L'échantillon a un volume total de 2 ml.

Cette boîte est ensuite placée sur une coupelle en aluminium et recouverte d'une cellule en quartz afin d'être exposée sous les U.V.A. (365 nm) à la dose d'1 J/cm2. L'ensemble composé de N.A.D.H., de riboflavine, de chlorure ferrique et d'E.D.T.A., soumis à l'exposition des U.V.A., va générer des espèces réduites de l'oxygène : $O_{2^\circ}^-$, $H_2O_2$, et principalement le radical hydroxyle °OH. Ce dernier va réagir avec la méthionine en libérant de l'éthylène dont la quantité est mesurée par chromatographie en phase gazeuse.

Plus la quantité de radicaux libres formés est grande, plus est importante la quantité d'éthylène libéré. Les résultats sont exprimés en pourcentage de pouvoir inhibiteur correspondant au pourcentage de diminution de la production d'éthylène par rapport au témoin (contenant 100 µl de tampon phosphate remplaçant le produit à tester).

Les substances étudiées sont introduites à l'aide d'une micropipette. Ce sont :

- la SOD (origine : SIGMA titrant 400 unités par mg),
- la chlorophylle A (origine : Sigma), la chlorophylline (origine : Wackherr) et l'hémoglobine bovine (origine : Sigma).

Conditions chromatographiques : (appareil de type VARIAN 3740)

- température injecteur : 80°C,
- température colonne : 80°C,
- température détecteur : 250°C,
- pression d'hélium : 36 psi (soit environ $2,4.10^5$ Pa),
- colonne : alumina F1 60/80 mesh (origine : Supelco),
- longueur : 2 m,
- diamètre externe : 1/8.

Les résultats (moyenne de 3 essais) sont résumés dans le tableau suivant :

| Echantillon | SOD Sigma (unités/ml d'echantillon) | Chlorophylle A % g/100 ml | Chlorophylline % g/100 ml | Hémoglobine % g/100 ml | % pouvoir inhibiteur | Augmentation du pouvoir inhibiteur |
|---|---|---|---|---|---|---|
| $E_1$ | 1 | - | - | - | 33,8 | - |
| $E_2$ | - | 0,015 | - | - | 21,4 | - |
| $E_3$ | 0,5 | 0,0075 | - | - | 60,1 | 78 % |
| $E_4$ | 50 | - | - | - | 55,8 | - |
| $E_5$ | - | - | 0,005 | - | 31,7 | - |
| $E_6$ | 25 | - | 0,0025 | - | 63,5 | 14 % |
| $E_7$ | 50 | - | - | - | 55,8 | - |
| $E_8$ | - | - | - | 0,025 | 42,1 | - |
| $E_9$ | - | - | - | 0,05 | 68,8 | - |
| $E_{10}$ | - | - | - | 0,075 | 76,3 | - |
| $E_{11}$ | 25 | - | - | 0,0125 | 76,1 | 28 % |
| $E_{12}$ | 25 | - | - | 0,025 | 80,1 | 16,4 % |
| $E_{13}$ | 25 | - | - | 0,0375 | 84,0 | 10,1 % |
| $E_{14}$ | 15 | - | - | - | 46,5 | - |
| $E_{15}$ | - | - | - | 0,025 | 42,1 | - |
| $E_{16}$ | 7,5 | - | - | 0,0125 | 61,9 | 33,1 % |

Les pourcentages indiqués sont en poids/volume. Une unité de SOD correspond à 2,5 µg.

Pour calculer l'augmentation du pouvoir inhibiteur de l'association, on a déterminé le pourcentage d'augmentation du pouvoir inhibiteur de l'association par rapport au pouvoir inhibiteur du composé de l'association qui, seul, donne le plus fort pourcentage de pouvoir inhibiteur.

L'étude de ces résultats par l'analyse de variance à 1 facteur montre qu'ils sont statistiquement significatifs.

L'échantillon $E_3$ montre qu'en associant la SOD et la chlorophylle A, on obtient un pouvoir inhibiteur bien supérieur à celui de la SOD seule de l'échantillon $E_1$, utilisée en une quantité double de celle utilisée dans l'association, ou à celui de la chlorophylle A seule de l'échantillon $E_2$, utilisée en une quantité double de celle utilisée dans l'association.

De même, l'échantillon $E_6$ montre qu'en associant la SOD et la chlorophylline, on obtient un pouvoir inhibiteur significativement supérieur à celui de la SOD seule de l'échantillon $E_4$, utilisée en une quantité double de celle utilisée dans l'association, ou à celui de la chlorophylline seule de l'échantillon $E_5$, utilisée en une quantité double de celle utilisée dans l'association.

Il en est de même des échantillons $E_{11}$ à $E_{13}$ et $E_{16}$ qui montrent que l'association de SOD et d'hémoglobine donne un pouvoir inhibiteur significativement supérieur à celui de la SOD seule des échantillons $E_7$ et $E_{14}$, utilisée en une quantité double de celle utilisée dans l'association, ou à celui de l'hémoglobine seule des échantillons $E_8$ à $E_{10}$ et $E_{15}$, utilisée en une quantité double de celle utilisée dans l'association.

**Revendications**

1. Composition cosmétique et/ou dermatologique, caractérisée par le fait qu'elle comprend au moins une superoxyde dismutase et au moins une porphyrine.

2. Composition cosmétique et/ou dermatologique selon la revendication 1, caractérisée par le fait que ladite superoxyde dismutase est choisie parmi les enzymes naturelles capables de catalyser la réaction de dismutation des ions superoxydes, et tout produit équivalent ayant une telle activité.

3. Composition cosmétique et/ou dermatologique selon l'une quelconque des revendications précédentes, caractérisée par le fait que la dite porphyrine est choisie parmi les porphyrines à transfert de charge.

4. Composition cosmétique et/ou dermatologique selon l'une quelconque des revendications précédentes, caractérisée par le fait que la dite porphyrine est choisie parmi la chlorophylle, la chlorophylline, l'hémoglobine et un extrait végétal ou animal contenant de la chlorophylle et/ou de l'hémoglobine.

5. Composition cosmétique et/ou dermatologique selon l'une quelconque des revendications précédentes, caractérisée par le fait que la concentration de la superoxyde dismutase est choisie dans la gamme de 40 à 5000 unités enzymatiques pour 100 g de composition.

6. Composition cosmétique et/ou dermatologique selon la revendication 5, caractérisée par le fait que la concentration de la superoxyde dismutase est choisie dans la gamme de 250 à 1500 unités enzymatiques pour 100 g de composition.

7. Composition cosmétique et/ou dermatologique selon l'une quelconque des revendications précédentes, caractérisée par le fait que la porphyrine est présente à raison de 0,001 % à 1 % en poids par rapport au poids total de la composition.

8. Composition cosmétique et/ou dermatologique selon la revendication 7, caractérisée par le fait que la porphyrine est présente à raison de 0,025 % à 0,1 % en poids par rapport au poids total de la composition.

9. Composition pour prévenir et/ou lutter contre le vieillissement cutané et/ou pour protéger la peau, les cheveux et/ou les muqueuses contre les effets nocifs et/ou inesthétiques provoqués par les radicaux libres, caractérisée en ce qu'elle consiste en une composition selon l'une quelconque des revendications 1 à 8.

10. Utilisation, en association, d'au moins une superoxyde dismutase et d'au moins une porphyrine comme ingrédients cosmétiques actifs dans ou pour la fabrication d'une composition cosmétique et/ou dermatologique destinée à prévenir et/ou lutter contre le vieillissement cutané et/ou à protéger la peau, les cheveux et/ou les muqueuses contre les effets nocifs et/ou inesthétiques provoqués par les radicaux libres.

11. Utilisation selon la revendication précédente, caractérisée par le fait que ladite composition est telle que définie dans l'une quelconque des revendications 1 à 8.

12. Procédé de traitement cosmétique, caractérisée par le fait que l'on applique sur la peau, sur les cheveux et/ou sur les muqueuses, une composition telle que définie dans l'une quelconque des revendications 1 à 8.

**Claims**

1. Cosmetic and/or dermatological composition, characterized in that it comprises at least one superoxide dismutase and at least one porphyrin.

2. Cosmetic and/or dermatological composition according to Claim 1, characterized in that the said superoxide dismutase is chosen from natural enzymes which are capable of catalysing the dismutation reaction of superoxide ions and any equivalent product having such an activity.

3. Cosmetic and/or dermatological composition according to either of the preceding claims, characterized in that the said porphyrin is chosen from charge transfer porphyrins.

4. Cosmetic and/or dermatological composition according to any one of the preceding claims, characterized in that the said porphyrin is chosen from chlorophyll, chlorophyllin, haemoglobin and a vegetable or animal extract containing chlorophyll and/or haemoglobin.

5. Cosmetic and/or dermatological composition according to any one of the preceding claims, characterized in that the concentration of the superoxide dismutase is chosen within the range from 40 to 5000 enzymatic units per 100 g of composition.

6. Cosmetic and/or dermatological composition according to Claim 5, characterized in that the concentration of the superoxide dismutase is chosen within the range from 250 to 1500 enzymatic units per 100 g of composition.

7. Cosmetic and/or dermatological composition according to any one of the preceding claims, characterized in that the porphyrin is present in a proportion of from 0.001 %-to 1-% by weight, relative to the total weight of the composition.

8. Cosmetic and/or dermatological composition according to Claim 7, characterized in that the porphyrin is present in a proportion of from 0.025 % to 0.1 % by weight, relative to the total weight of the composition.

9. Composition for preventing and/or combating skin ageing and/or for protecting the skin, hair and/or mucosae against the harmful and/or unaesthetic effects caused by free radicals, characterized in that it comprises a composition according to any one of Claims 1 to 8.

10. Use, in combination, of at least one superoxide dismutase and at least one porphyrin as cosmetic active ingredients in or for the manufacture of a cosmetic and/or dermatological composition which is intended for preventing and/or combating skin ageing and/or for protecting the skin, hair and/or mucosae against the harmful and/or unaesthetic effects caused by free radicals.

11. Use according to the preceding claim, characterized in that the said composition is as defined in any one of Claims 1 to 8.

12. Method of cosmetic treatment, characterized in that a composition as defined in any one of Claims 1 to 8 is applied to the skin, hair and/or mucosae.

**Patentansprüche**

1. Kosmetische und/oder dermatologische Zusammensetzung, **dadurch gekennzeichnet, daß** sie mindestens eine Superoxid-Dismutase und mindestens ein Porphyrin enthält.

2. Kosmetische und/oder dermatologische Zusammensetzung nach Anspruch 1, dadurch gekennzeichnet, daß die Superoxid-Dismutase unter natürlichen Enzymen, welche die Disproportionierungsreaktion von Hyperoxid-Radikalionen katalysieren können, und beliebigen äquivalenten Stoffen mit dieser Wirksamkeit ausgewählt ist.

3. Kosmetische und/oder dermatologische Zusammensetzung nach Anspruch 1 oder 2, dadurch gekennzeichnet, daß das Porphyrin unter den Porphyrinen mit Ladungsübertragung ausgewählt ist.

4. Kosmetische und/oder dermatologische Zusammensetzung nach einem der vorhergehenden Ansprüche, dadurch gekennzeichnet, daß das Porphyrin unter Chlorophyll, Chlorophyllin, Hämoglobin und pflanzlichen oder tierischen Extrakten, die Chlorophyll und/oder Hämoglobin enthalten, ausgewählt ist.

5. Kosmetische und/oder dermatologische Zusammensetzung nach einem der vorhergehenden Ansprüche, dadurch gekennzeichnet, daß die Konzentration der Superoxid-Dismutase im Bereich von 40 bis 5000 Enzymeinheiten pro 100 g Zusammensetzung gewählt ist.

6. Kosmetische und/oder dermatologische Zusammensetzung nach Anspruch 5, dadurch gekennzeichnet, daß die Konzentration der Superoxid-Dismutase im Bereich von 250 bis 1500 Enzymeinheiten pro 100 g Zusammensetzung gewählt ist.

7. Kosmetische und/oder dermatologische Zusammensetzung nach einem der vorhergehenden Ansprüche, dadurch gekennzeichnet, daß das Porphyrin in einem Mengenanteil von 0,001 bis 1 Gew.-%, bezogen auf das Gesamtgewicht der Zusammensetzung, vorliegt.

8. Kosmetische und/oder dermatologische Zusammensetzung nach Anspruch 7, dadurch gekennzeichnet, daß das Porphyrin in einem Mengenanteil von 0,025 bis 0,1 Gew.-%, bezogen auf das Gesamtgewicht der Zusammensetzung, vorliegt.

9. Zusammensetzung, um Hautalterung vorzubeugen und/oder sie zu bekämpfen und/oder die Haut, die Haare und/oder die Schleimhäute gegen schädliche und/oder unästhetische Wirkungen zu schützen, die durch freie Radikale hervorgerufen werden, dadurch gekennzeichnet, daß sie aus einer Zusammensetzung nach einem der Ansprüche 1 bis 8 besteht.

10. Verwendung mindestens einer Superoxid-Dismutase in Kombination mit mindestens einem Porphyrin als kosmetische Wirkstoffe in oder zur Herstellung von kosmetischen und/oder dermatologischen Zusammensetzungen, um Hautalterung vorzubeugen und/oder sie zu bekämpfen und/oder die Haut, die Haare und/oder die Schleimhäute gegen schädliche und/oder unästhetische Wirkungen zu schützen, die durch freie Radikale hervorgerufen werden.

11. Verwendung nach Anspruch 10, dadurch gekennzeichnet, daß die Zusammensetzung eine Zusammensetzung nach einem der Ansprüche 1 bis 8 ist.

12. Verfahren zur kosmetischen Behandlung, dadurch gekennzeichnet, daß eine Zusammensetzung nach einem der Ansprüche 1 bis 8 auf die Haut, die Haare und/oder die Schleimhäute aufgebracht wird.